# EUROPÄISCHE PATENTSCHRIFT

(11) **EP 4 092 407 B1**
(45) Veröffentlichungstag und Bekanntmachung des Hinweises auf die Patenterteilung: **18.10.2023**
(21) Anmeldenummer: 22170318.4
(22) Anmeldetag: 27.04.2022
(51) Int. Cl.: G01N 21/90, G01N 21/954

(54) **INSPEKTIONSVORRICHTUNG ZUR INSPEKTION VON ZYLINDRISCHEN METALLFORMTEILEN**
INSPECTION DEVICE FOR INSPECTING CYLINDRICAL FORMED METAL PARTS
DISPOSITIF D'INSPECTION POUR L'INSPECTION DE PIÈCES MÉTALLIQUES CYLINDRIQUES FORMÉES

(30) Priorität: 04.05.2021 DE 102021111517
(43) Veröffentlichungstag der Anmeldung: 23.11.2022
(73) Patentinhaber: H & T Marsberg GmbH & Co. KG, 34431 Marsberg (Bredelar) (DE)
(72) Erfinder: von Rüden, Gregor, 34431 Marsberg (DE); Kinold, Martin, 34431 Marsberg (DE); Götte, Tobias, 34431 Marsberg (DE); Rosenkranz, Daniel, 34431 Marsberg (DE)
(74) Vertreter: Uexküll & Stolberg

(56) Entgegenhaltungen:
- EP-A2- 3 078 960
- DE-A1-102005 053 537
- DE-A1-102014 106 235

## Beschreibung

### TECHNISCHES GEBIET

Die vorliegende Erfindung betrifft eine Inspektionsvorrichtung zur Inspektion von zylindrischen Metallformteilen

### HINTERGRUND DER ERFINDUNG

Zylindrische Metallformteile wie beispielsweise Batteriebecher oder Aerosolbehälter für Dosierinhalatoren werden typischerweise mittels Tiefziehen hergestellt. Dazu wird eine Ronde aus einem Blech ausgestanzt, zu einem Napf gezogen und dann in mehreren Tiefziehschritten mit Hilfe eines Stempels und einer Matrize in ein Metallformteil, insbesondere in einen Batteriebecher oder einen Aerosolbehälter tiefgezogen.

Weist das Blech beispielsweise produktionsbedingte Schlackeeinschlüsse auf, so können diese Schlackeeinschlüsse zu Oberflächenfehlern, beispielsweise parabolischen Aufbrüchen, Löchern oder ähnlichen Ziehfehlern, im tiefgezogenen Metallformteil führen. Darüber hinaus können Verunreinigungen, beispielsweise Metallspäne, in der Matrize ebenfalls zu Oberflächenfehlern, insbesondere Löchern, in den Metallformteilen führen. Es ist daher notwendig, die Metallformteile nach ihrer Produktion auf herstellungsbedingte Fehlstellen zu kontrollieren. Diese Kontrolle erfolgt derzeit manuell. Ein Mitarbeiter prüft stichprobenartig einzelne Metallformteile auf Fehlstellen. Weisen einzelne Metallformteile Fehlstellen auf, so muss die gesamte Charge kontrolliert werden. Dies führt zu erhöhten Kosten, da insbesondere die Nachkontrolle gesamter Produktionschargen extern durch Dienstleister durchgeführt wird.

Aus dem Stand der Technik sind Inspektionsvorrichtungen bekannt, die als Teil einer Produktionslinie zur Überwachung der Qualität der gefertigten Produkte eingesetzt werden.

Die DE 10 2004 052 508 A1 betrifft ein System zum Vermessen eines Körpers und zur Überwachung der Oberfläche des Körpers mit Hilfe einer Kameraeinrichtung. Das System wird zur Kontrolle von Endlosprofilen mit Hilfe elektronisch digitaler Bildverarbeitung verwendet. Ein Bestrahlen der Profiloberfläche mittels einer geeigneten Lichtquelle und ein Detektieren der reflektierten Strahlung durch eine elektronische Kameraeinrichtung mit anschließender elektronischer Bildverarbeitung gewährt Rückschlüsse auf die Abmessung und Toleranzen eines Körpers als auch über die Beschaffenheit seiner Oberfläche.

DE 10 2014 106235 A1 offenbart eine Inspektionsvorrichtung zur Inspektion von zylindrischen Metallformteilen umfassend eine Führung zum Führen der zu inspizierenden Metallformteile durch die Inspektionsvorrichtung, Strahlungsmittel, die ausgebildet sind, Strahlung in Richtung der geführten Metallformteile abzugeben, so dass die Strahlung auf jedes der Metallformteile trifft und von diesem zumindest teilweise als Reflexionsstrahlung reflektiert wird, eine Sensoreinrichtung, die ausgebildet ist, die Reflexionsstrahlung zu empfangen und zu Bilddaten umzuwandeln, und eine Auswerteeinrichtung, die ausgebildet ist, basierend auf einer Auswertung der Bilddaten zu beurteilen, ob das jeweilige Metallformteil herstellungsbedingte Fehlstellen aufweist.

Für eine kontinuierlich zuverlässige Qualitätskontrolle der produzierten Metallformteile ist es notwendig, dass diese in einer annähernd gleichbleibenden Lage relativ zu der Inspektionsvorrichtung, beispielsweise der Kameraeinrichtung, vorbeigeführt werden. Dies kann bei Endlosprofilen ohne weiteres realisiert werden. Bei einzelnen kleineren Produkten, wie beispielsweise Batteriebechern oder Aerosolbehältern, jedoch, insbesondere bei hohen Taktzahlen von bis zu 400 Metallformteilen pro Minute, ist dies bisher nicht möglich.

Es ist daher Aufgabe der vorliegenden Erfindung, eine Inspektionsvorrichtung zur Inspektion von zylindrischen Metallformteilen bereitzustellen, die eine Inspektion von einzelnen Metallformteilen bei hohen Taktzahlen ermöglicht.

### ZUSAMMENFASSUNG DER ERFINDUNG

Die Aufgabe wird von einer Inspektionsvorrichtung umfassend die Merkmale gemäß Schutzanspruch 1 gelöst. Bevorzugte Ausführungsformen sind in den Unteransprüchen beschrieben.

Gemäß der vorliegenden Erfindung umfasst die Inspektionsvorrichtung eine Führung zum Führen der zu inspizierenden Metallformteile durch die Inspektionsvorrichtung. Die Inspektionsvorrichtung umfasst weiter Strahlungsmittel, die ausgebildet sind, Strahlung in Richtung der geführten Metallformteile abzugeben, so dass die Strahlung auf jedes der Metallformteile trifft und von diesen zumindest teilweise als Reflexionsstrahlung reflektiert wird. Bevorzugt handelt es sich bei den Strahlungsmitteln um Lichtquellen und bei der ausgesendeten Strahlung um Licht mit einer Wellenlänge im Bereich vom 400 nm bis 800 nm.

Die Inspektionsvorrichtung umfasst weiter eine Sensoreinrichtung, die ausgebildet ist, die Reflexionsstrahlung zu empfangen und zu Bilddaten umzuwandeln. Optional ist die Sensoreinrichtung eine Kameraeinrichtung. Weiter ist eine Auswerteeinrichtung Teil der erfindungsgemäßen Inspektionsvorrichtung. Die Auswerteeinrichtung ist ausgebildet, basierend auf einer Auswertung der Bilddaten zu beurteilen, ob das jeweilige Metallformteil herstellungsbedingte Fehlstellen aufweist. Optional erfolgt die Bildauswertung computergestützt mithilfe einer entsprechenden Software.

Erfindungsgemäß wird die Führung durch ein Führungsrohr gebildet. Das Führungsrohr ist konfiguriert, die zu inspizierenden Metallformteile aufzunehmen, so dass diese in dem Führungsrohr durch die Inspektionsvorrichtung geführt werden. Das Führungsrohr weist einen Transmissionsgrad für die Strahlung von 80 % bis 95 % auf, gemessen bei einer Wanddicke des Führungsrohrs von 2 mm und für Strahlung aus einem Wellenlängenbereich von 400 nm bis 800 nm. Das Führungsrohr hat einen mittleren linearen thermischen Ausdehnungskoeffizient nach ISO 7991 von höchstens 5×10⁻⁶/K. Bevorzugt weist das Führungsrohr eine Brechzahl von 1,4 bis 1,5, insbesondere von 1,473 auf.

Durch die Verwendung eines Führungsrohrs wird es ermöglicht, einzelne kleine Metallformteile, beispielsweise mit den Abmessungen eines Batteriebechers, automatisch, d.h. ohne manuelle Arbeitsschritte, durch die Inspektionsvorrichtung zu führen. Dadurch wird gewährleistet, dass die Metallformteile immer in gleicher Lage relativ zu den Strahlungsmitteln und der Sensoreinrichtung vorbeibewegt werden. Produktionsbedingte Fehlstellen können dadurch sicher erkannt werden. Darüber hinaus ermöglicht ein Transmissionsgrad des Führungsrohrs zwischen 80 % und 95 %, dass die von den Strahlungsmitteln ausgesendete Strahlung durch das Führungsrohr hindurchtreten kann und auf die zu inspizierenden Metallformteile trifft. Der genannte Transmissionsgrad ermöglicht weiter, dass die von den zu inspizierenden Metallformteilen reflektierte Strahlung erneut durch das Führungsrohr hindurch treten kann und auf die Sensoreinrichtung trifft. Der mittlere lineare thermische Ausdehnungskoeffizient von höchstens 5-10⁻⁶/K bewirkt eine Temperaturbeständigkeit, so dass eine eventuelle durch die Strahlungsmittel verursachte Erwärmung des Führungsrohrs vernachlässigbare Schäden bzw. Verformungen des Führungsrohrs verursacht. Dadurch wird eine Reflexion der Strahlung an den Metallformteilen, wenn überhaupt, nur auf eine zu vernachlässigende Art und Weise negativ beeinflusst.

Gemäß einer Ausführungsform der Erfindung weist das Führungsrohr einen Transmissionsgrad von 80 % bis 95 %, gemessen auch bei einer Wanddicke des Führungsrohrs von 4 mm und für Strahlung aus einem Wellenlängenbereich von 400 nm bis 800 nm, auf.

Gemäß einer weiteren Ausführungsform der Erfindung weist das Führungsrohr eine Knoop'sche Härte HK_{0.1/20} gemäß ISO 9385 von 200 bis 700 auf. Die Härte des Führungsrohrs verhindert, dass die Metallformteile das Führungsrohr oberflächlich beschädigen, beispielsweise in Form von Riefen, Kratzer oder durch Abrieb, und dadurch trüben. Die Härte des Führungsrohrs gewährlistet somit, dass der anspruchsgemäße Transmissionsgrad von 80% bis 95% während der Nutzung des Führungsrohrs vorliegt und nicht durch Beschädigungen durch die Metallformteile herabgesetzt wird.

Bevorzugt ist das Führungsrohr aus Borosilicatglas hergestellt. Borosilicatglas ist besonders robust, stoßfest und schnittfest. Es ist hitzebeständig und kann daher auch bei hohen Temperaturen eingesetzt werden. Es hält starken Temperaturschwankungen stand und verfügt darüber hinaus über eine hohe chemische Beständigkeit.

Gemäß einer weiteren Ausführungsform ist das Borosilicatglas ein erdalkalifreies Borosilicatglas. Erdalkalifreies Borosilicatglas wird auch Borosilicatglas 3.3 genannt. Bevorzugt wird für das Führungsrohr das Borosilicatglas 3.3 mit der Bezeichnung Duran^{®} der Firma Schott AG, 55122 Mainz verwendet. Borosilicatglas 3.3 entspricht DIN ISO 3585, ist chemisch hoch resistent, hat eine sehr gute Temperaturwechselbeständigkeit und ermöglicht dadurch eine vielseitige Anwendung, beispielsweise im Glasapparatebau, bei Laborartikeln, und für kunstgewerbliche Zwecke. Duran^{®} verfügt über eine ausreichende Widerstandsfähigkeit für den kontinuierlichen Transport von Metallformteilen. Die durch das Führungsrohr bewegten Metallformteile verursachen keine Trübung des Führungsrohrs, beispielsweise durch Abrieb oder Beschädigung des Führungsrohrs. Die Bestrahlung des Führungsrohrs durch die Strahlungsmittel und die damit erhöhte Temperatur führt zu einer vernachlässigbare Verformung oder vernachlässigbaren Beschädigung des Führungsrohrs.

Bevorzugt weist das Führungsrohr einen Innendurchmesser und die zylindrischen Metallformteile jeweils einen Außendurchmesser auf. Der Außendurchmesser der zylindrischen Metallformteile ist kleiner als der Innendurchmesser des Führungsrohrs. Die Differenz zwischen dem Innendurchmesser des Führungsrohrs und dem Außendurchmesser der Metallformteile beträgt zwischen 1 mm und 6 mm, bevorzugt zwischen 2 mm und 5 mm, insbesondere bevorzugt zwischen 2,3 mm und 4,8 mm. Die beschriebenen Differenzen zwischen dem Innendurchmesser des Führungsrohrs und dem Außendurchmesser der Metallformteile ermöglicht ein Bewegen der Metallformteile durch das Führungsrohr ohne Verkanten oder Verklemmen. Gleichzeitig ermöglichen die beschriebenen Differenzen eine gleichbleibende Lage der Metallformteile relativ zu der Inspektionsvorrichtung während sie sich durch das Führungsrohr bewegen. Bevorzugt bildet der Innendurchmesser des Führungsrohrs über die Länge des Führungsrohrs einen Hohlraum aus, durch den die Metallformteile geführt werden.

Gemäß einer Ausführungsform erstreckt sich das Führungsrohr entlang einer Längsachse durch die Inspektionsvorrichtung. Dadurch werden Hindernisse, die beispielsweise durch einen kurvenförmigen Verlauf des Führungsrohrs entstehen würden, vermieden. Ein ungehindertes Bewegen der Metallformteile durch das Führungsrohr ist damit gewährleistet.

Gemäß einer weiteren Ausführungsform weist die Inspektionsvorrichtung eine Aufnahme für das Führungsrohr auf. Die Inspektionsvorrichtung ist derart ausgebildet, dass das Führungsrohr aus der Aufnahme entnehmbar und austauschbar ist. Dadurch kann das Führungsrohr beispielsweise bei einer Beschädigung einfach aus der Inspektionsvorrichtung entnommen und ausgetauscht werden. Bevorzugt weist die Inspektionsvorrichtung einen Mechanismus zum manuellen Öffnen der Inspektionsvorrichtung auf. Durch Betätigung des Mechanismus kann die Inspektionsvorrichtung geöffnet werden, wodurch die Aufnahme durch das Führungsrohr frei zugänglich ist. Bevorzugt kann das Führungsrohr manuell aus der Aufnahme entnommen werden.

Bevorzugt hat das Führungsrohr einen Außendurchmesser. Optional liegt der Außendurchmesser des Führungsrohres in einem Bereich von 20 mm bis 50 mm.

Die Aufnahme ist ausgebildet, Führungsrohre mit unterschiedlichen Außendurchmessern aufzunehmen. Optional kann die Inspektionsvorrichtung Metallformteile unterschiedlicher Außendurchmesser inspizieren. Hierzu ist es notwendig, unterschiedliche Führungsrohre zu verwenden, die in ihrem Innendurchmesser auf den entsprechenden Außendurchmesser des zu untersuchenden Metallformteils abgestimmt sind. Um eine ausreichende Festigkeit der Führungsrohre bei unterschiedlichen Innendurchmessern zu gewährleisten, weisen die Führungsrohre unterschiedliche Außendurchmesser auf. Indem die Aufnahme Führungsrohre mit unterschiedlichen Außendurchmessern aufnehmen kann, wird eine flexiblere Verwendung der Inspektionsvorrichtung für unterschiedlich dimensionierte Metallformteile ermöglicht. Bevorzugt weist die Aufnahme mindestens zwei einander gegenüberliegende Anschläge auf, die relativ zueinander verschiebbar an der Inspektionsvorrichtung befestigt sind. Bevorzugt wird jeder Anschlag durch eine Stirnseite eines Körpers gebildet, wobei das Führungsrohr zwischen den Stirnseiten einklemmbar ist. Optional umfasst die Aufnahme vier Anschläge, wobei jeweils zwei einander gegenüberliegenden Anschläge ein Paar bilden und die beiden Paare entlang der Längsachse des Führungsrohrs beabstandet voneinander angeordnet sind. Die beiden Paare sind beispielsweise außerhalb eines Gehäuses der Inspektionsvorrichtung entlang der Längsachse des Führungsrohrs vor und hinter dem Gehäuse der Inspektionsvorrichtung angeordnet.

Gemäß einer Ausführungsform weist das Führungsrohr eine Wanddicke von 2 mm bis 4 mm auf. Bevorzugt hat das Führungsrohr eine Wanddicke von 2 mm bis 3 mm bei einem Außendurchmesser der zu inspizierenden Metallformteile zwischen 14 mm und 17 mm. Weiter bevorzugt hat das Führungsrohr eine Wanddicke von 3 mm bis 4 mm bei einem Außendurchmesser der zu inspizierenden Metallformteile von 25 mm bis 33 mm. Bevorzugt beträgt der Transmissionsgrad des Führungsrohrs zwischen 80 % und 95 % für Strahlung in einem Wellenlängenbereich von 400 nm bis 800 nm bei einer Wanddicke des Führungsrohrs von 2 mm bis 4 mm. Dadurch ist bei der Verwendung unterschiedlicher Wanddicken eine annähernd gleichbleibende Transmission der Strahlung der Strahlungsmittel sowie der Reflexionsstrahlung durch das Führungsrohr gewährleistet. Bei der Verwendung unterschiedlicher Wanddicken trifft ein annähernd gleichbleibendes Maß an Reflexionsstrahlung auf die Sensoreinrichtung, so dass eine zuverlässige Erkennung von Fehlstellen durch die Auswerteeinrichtung ermöglicht wird.

Bevorzugt ist das Führungsrohr derart angeordnet, dass sich die Metallformteile mittels Schwerkraft durch das Führungsrohr bewegen. Optional ist das Führungsrohr relativ zum Boden geneigt, sodass sich entlang des Führungsrohres ein Gefälle ergibt. Bevorzugt werden die zu inspizierenden Metallformteile mit einem Förderband an ein oberes Ende des Führungsrohrs herangeführt und fallen mittels Schwerkraft durch das Führungsrohr hindurch. Dadurch sind für das Bewegen der zu inspizierenden Metallformteile innerhalb des Führungsrohres keine zusätzlichen unterstützenden Maßnahmen notwendig.

Gemäß einer Ausführungsform der Erfindung ist das Führungsrohr mit Druckluft beaufschlagbar und derart ausgebildet, dass die Metallformteile mittels Druckluftstößen durch das Führungsrohr bewegt werden. Ist beispielsweise die Inspektionsvorrichtung derart angeordnet, dass das Führungsrohr beispielsweise waagerecht oder annähernd waagerecht positioniert ist, so können die zu inspizierenden Metallformteile mit einzelnen Druckluftstößen durch das Führungsrohr bewegt werden. Dadurch kann die Inspektionsvorrichtung auch an Orten verwendet werden, an denen eine geneigte Position des Führungsrohrs nicht möglich ist.

Gemäß einer weiteren Ausführungsform der Erfindung sind die Metallformteile Edelstahlbecher, insbesondere Batteriebecher. Batteriebecher werden bevorzugt aus Stahl, insbesondere Edelstahl hergestellt und weisen eine im Wesentlichen zylindrische Form auf.

Die oben genannte Aufgabe wird ebenfalls von einer Produktionsanlage umfassend die Merkmale gemäß Patentanspruch 12 gelöst.

Erfindungsgemäß umfasst die Produktionsanlage zum Herstellen von zylindrischen Metallformteilen eine Tiefziehpresse zum Tiefziehen der Metallformteile aus Rohlingen in mehreren Tiefziehschritten mit Hilfe eines Stempels und einer Matrize. Die Produktionsanlage umfasst weiter eine Waschstation zum Reinigen der tiefgezogenen Metallformteile und eine oben beschriebene Inspektionsvorrichtung zur Inspektion der gereinigten Metallformteile. Mit Hilfe der Inspektionsvorrichtung ist es möglich, kontinuierlich tiefgezogene und gereinigte Metallformteile zu inspizieren. Bevorzugt ist die Inspektionsvorrichtung ausgebildet, bis zu 400 Metallformteile pro Minute zu inspizieren. Optional wird die Taktrate, in der die Metallformteile durch die Inspektionsvorrichtung geführt werden durch die Taktrate der Waschstation, d.h. wie viele Metallformteile pro Minute die Waschstation verlassen, bestimmt.

Die oben genannte Aufgabe wird ebenfalls von einem Verfahren zum Inspizieren von zylindrischen Metallformteilen gelöst. Erfindungsgemäß umfasst das Verfahren die folgenden Schritte:
Bereitstellen einer erfindungsgemäßen Inspektionsvorrichtung;
Einbringen eines Metallformteils in das Führungsrohr, so dass dieses in dem Führungsrohr (11) durch die Inspektionsvorrichtung (9) geführt wird;
Aussenden von Strahlung, so dass diese auf das Metallformteil (2) trifft und von diesem zumindest teilweise als Reflexionsstrahlung reflektiert wird;
Empfangen der Reflexionsstrahlung und Umwandlung derselben zu Bilddaten;
Auswerten der Bilddaten und Beurteilen, ob das Metallformteil (2) herstellungsbedingte Fehlstellen aufweist.
BESCHREIBUNG DER ERFINDUNG

Die Erfindung wird im Folgenden anhand einer beispielhaften Ausführungsform beschrieben, die in den Figuren dargestellt ist, in denen:
- Figur 1: eine schematische Darstellung einer Produktionsanlage mit einer erfindungsgemäßen Inspektionsvorrichtung,
- Figur 2: eine schematische Darstellung der erfindungsgemäßen Inspektionsvorrichtung und
- Figur 3: eine Detailansicht der Inspektionsvorrichtung aus Figur 2 zeigt.

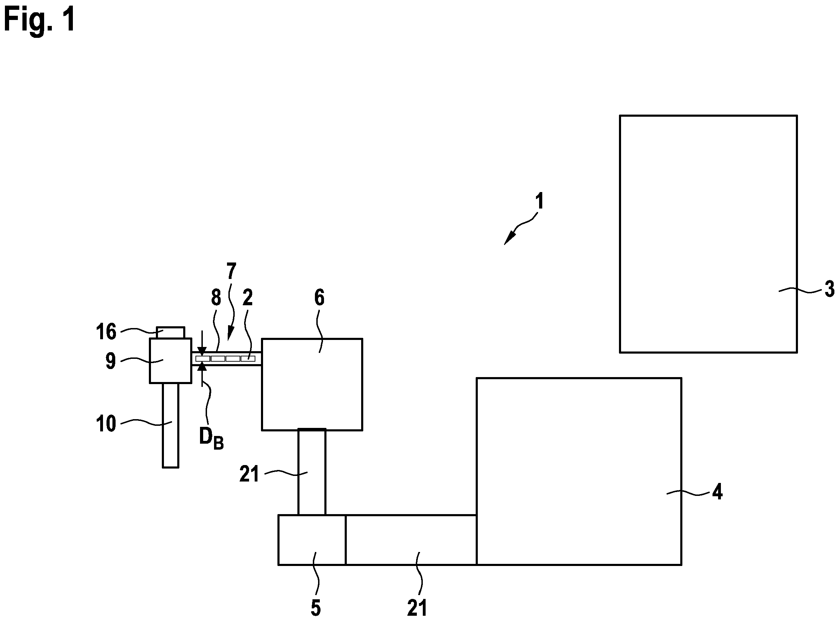

Figur 1 zeigt eine Produktionsanlage 1 zum Herstellen von zylindrischen Metallformteilen in Form von Batteriebechern 2. Die Produktionsanlage 1 umfasst eine Tiefziehpresse 3 zum Tiefziehen der Batteriebecher 2 aus einer Ronde, also einem runden Blechzuschnitt, in mehreren Tiefziehschritten mit Hilfe eines Stempels und einer Matrize. Optional kann der Tiefziehpresse 3 eine erste Station vorgeschaltet sein, in der aus dem Blechzuschnitt ein Napf gezogen wird. Der Napf wird dann in mehreren Tiefziehschritten in der Tiefziehpresse 3 mit Hilfe eines Stempels in einer Matrize zum Batteriebecher 2 tiefgezogen.

Die Produktionsanlage 1 umfasst weiter eine Waschanlage 4 zum Reinigen der tiefgezogenen Batteriebecher 2. Die Tiefziehpresse 3 ist von der Waschanlage 4 beabstandet angeordnet. Batteriebecher 2, die aus der Tiefziehpresse 3 ausgeworfen werden, werden manuell, beispielsweise in Boxen oder Kartonage, zur Waschanlage 4 transportiert.

Die Produktionsanlage 1 umfasst weiter eine Separierungseinrichtung 6 in Form eines Zentrifugalförderers. Die Separierungseinrichtung 6 ist ausgebildet, die gewaschenen Batteriebecher 2 zu vereinzeln und einem Zuführsystem 7 zuzuführen. Zwischen der Waschanlage 4 und der Separierungseinrichtung 6 ist ein Zwischenpuffer 5 in Form eines Bunkers angeordnet, der mit der Waschanlage 4 und der Separierungseinrichtung 6 über jeweils eine Fördereinrichtung 21, beispielsweise einen Rollenförderer oder einen Stollengutförderer verbunden ist.

Das Zuführsystem 7 weist ein oder mehrere Förderbänder 8 auf, mittels derer die Batteriebecher 2 einer Inspektionsvorrichtung 9 zur Inspektion der gereinigten Batteriebecher 2 zugeführt werden. Das Zuführsystem 7 ist ausgebildet, die Orientierung der aus der Separierungseinrichtung 6 austretenden Batteriebecher 2 zu überprüfen und falsch orientierte Batteriebecher 2 auszusondern.

An die Inspektionsvorrichtung 9 angrenzend ist eine Rollenbahn 10 angeordnet. Die Rollenbahn 10 ist ausgebildet, Paletten mit darauf befindlicher Kartonage zu transportieren. In der Kartonage sind die inspizierten und für gut befundenen Batteriebecher 2 angeordnet, die die Inspektionsvorrichtung 9 verlassen.

Ebenfalls an die Inspektionsvorrichtung 9 angrenzend ist ein Behältnis 16 zur Aufnahme von fehlerbehafteten Batteriebechern 2 angeordnet. Die fehlerbehafteten Batteriebecher 2 werden nicht in der Kartonage auf der Rollenbahn 10 gesammelt, sondern vorher automatisch über eine mechanische Weiche von den übrigen Batteriebechern 2 getrennt und in dem Behältnis 16 gesammelt.

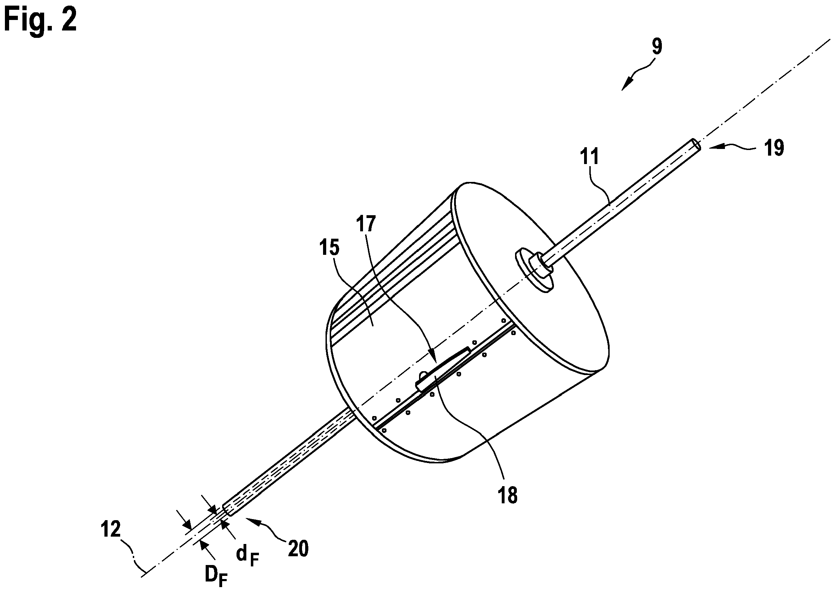

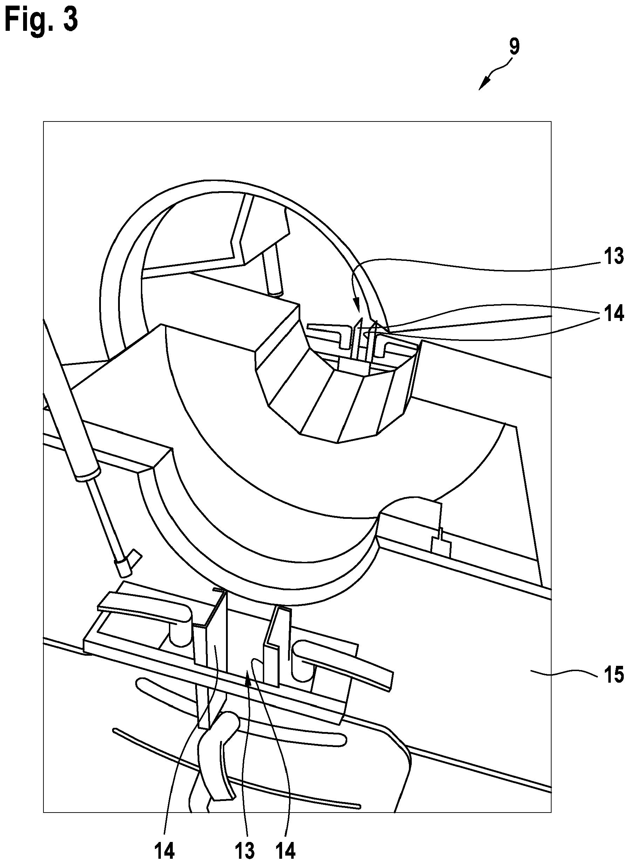

In den Figuren 2 und 3 ist die Inspektionsvorrichtung 9 im Detail dargestellt. Die Inspektionsvorrichtung 9 umfasst eine als Führungsrohr 11 ausgebildete Führung. Das Führungsrohr ist konfiguriert, um die zu inspizierenden Batteriebecher 2 aufzunehmen, so dass diese in dem Führungsrohr 11 durch die Inspektionsvorrichtung 9 geführt werden. Das Führungsrohr 11 hat ein erstes in Fig. 2 oberes Ende 19 zum Aufnehmen der Batteriebecher 2 und ein zweites in Fig. 2 unteres Ende 20, an dem die Batteriebecher 2 das Führungsrohr 11 verlassen.

Die Inspektionsvorrichtung 9 umfasst weiter Strahlungsmittel (nicht dargestellt), die ausgebildet sind, Strahlung in Richtung der geführten Batteriebecher 2 abzugeben, so dass die Strahlung auf jeden der Batteriebecher 2 trifft und von diesen zumindest teilweise als Reflexionsstrahlung reflektiert wird. Optional ist die von den Strahlungsmitteln abgegebene Strahlung sichtbares Licht mit einer Wellenlänge im Bereich von 400 nm bis 800 nm.

Die Inspektionsvorrichtung 9 umfasst ebenfalls eine Sensoreinrichtung (nicht dargestellt), die ausgebildet ist, die Reflexionsstrahlung zu empfangen und zu Bilddaten umzuwandeln. Optional ist die Sensoreinrichtung eine Kameraeinrichtung mit einem oder mehreren Kamerasystemen.

Die Inspektionsvorrichtung 9 umfasst eine Auswerteeinrichtung (nicht dargestellt), die ausgebildet ist, basierend auf einer Auswertung der Bilddaten zu beurteilen, ob der jeweilige Batteriebecher 2 herstellungsbedingte Fehlstellen wie bspw. parabolischen Aufbrüchen, Löcher oder sonstige Beschädigungen aufweist. Optional erfolgt die Bildauswertung computergestützt mithilfe einer entsprechenden Software.

Das Führungsrohr 11 ist aus erdalkalifreiem Borosilicatglas (Borosilicatglas 3.3 nach DIN ISO 3585) hergestellt. Bevorzugt ist das Führungsrohr 11 aus Borosilicatglas 3.3 mit der Handelsbezeichnung Duran^{®} der Firma Schott AG hergestellt. Das Führungsrohr 11 hat einen Transmissionsgrad für die Strahlung von 80 % bis 95 %, gemessen bei einer Wanddicke des Führungsrohrs von 2 mm und für Strahlung aus einem Wellenlängenbereich von 400 nm bis 800 nm. Das Führungsrohr 11 weist einen mittleren linearen thermischen Ausdehnungskoeffizient nach ISO 7991 von höchstens 5×10⁻⁶/K auf.

Das Führungsrohr 11 hat einen Innendurchmesser d_{F} sowie einen Außendurchmesser D_{F} und die zylindrischen Batteriebecher 2 jeweils einen Außendurchmesser D_{B} (siehe Figuren 1 und 2). Der Innendurchmesser d_{F} des Führungsrohrs 11 bildet über die Länge des Führungsrohrs 11 einen Hohlraum aus, durch den die Batteriebecher 2 geführt werden. Der Außendurchmesser D_{B} der Batteriebecher 2 ist kleiner als der Innendurchmesser d_{F} des Führungsrohrs 11. Die Differenz zwischen dem Innendurchmesser d_{F} des Führungsrohrs 11 und dem Außendurchmesser D_{B} der Batteriebecher 2 beträgt zwischen 2,3 mm und 4,8 mm. Das Führungsrohr 11 weist eine Wanddicke von 2 bis 4 mm auf.

Das Führungsrohr 11 erstreckt sich entlang einer Längsachse 12 durch die Inspektionsvorrichtung 9. Das Führungsrohr 11 ist derart angeordnet, dass sich die Batteriebecher 2 mittels Schwerkraft durch das Führungsrohr 11 bewegen. Hierzu ist das Führungsrohr gekippt, d.h. schräg zur Bodenaufstandsfläche der Inspektionsvorrichtung 9 angeordnet. Alternativ ist es ebenso möglich, das Führungsrohr 1 waagerecht oder annähernd waagerecht zu positionieren. In diesem Fall wird das Führungsrohr 11 mit Druckluft beaufschlagt. Es ist derart ausgebildet, dass die Batteriebecher 2 mittels Druckluftstößen oder mittels eines kontinuierlichen Luftstroms durch das Führungsrohr 11 bewegt werden können.

Die Inspektionsvorrichtung 9 hat eine Aufnahme 13 für das Führungsrohr 11. Die Aufnahme 13 ist ausgebildet, Führungsrohre 11 mit unterschiedlichen Außendurchmessern aufzunehmen. Die Aufnahme 13 umfasst vier Anschläge 14, wobei jeweils zwei der Anschläge 14 einander gegenüberliegend angeordnet sind und jeweils zwei Anschläge 14 ein Paar bilden. Die beiden Anschläge 14 eines Paares sind relativ zueinander verschiebbar und in unterschiedlichen Abständen zueinander arretierbar auf der Inspektionsvorrichtung 9 angeordnet. Die beiden Paare sind entlang der Längsachse 12 des Führungsrohrs 11 voneinander beabstandet positioniert. Die beiden Paare sind außerhalb eines Gehäuses 15 der Inspektionsvorrichtung 9 entlang der Längsachse 12 des Führungsrohrs 11 vor und hinter dem Gehäuse 15 der Inspektionsvorrichtung 9 angeordnet.

Die Inspektionsvorrichtung 9 ist derart ausgebildet, dass das Führungsrohr 11 aus der Aufnahme 13 entnehmbar und austauschbar ist. Hierzu weist die Inspektionsvorrichtung 9 einen manuell betätigbaren Mechanismus 17 mit einem drehbaren Griff 18 auf. Durch Drehen des Griffs 18 kann die Inspektionsvorrichtung 9, wie in Figur 3 gezeigt, geöffnet werden, so dass die Aufnahme 13 für das Führungsrohr 11 zugänglich ist.

Im Folgenden wird ein Verfahren zum Inspizieren von zylindrischen Metallformteilen unter Bezugnahme auf die Figuren 1 bis 3 beschrieben.

Die Batteriebecher 2 werden über ein Förderband 8 des Zuführsystems 7 an das obere Ende 19 des Führungsrohrs 11 der Inspektionsvorrichtung 9 befördert. Mittels des Fördersystems 7 wird sichergestellt, dass die Batteriebecher 2 das Führungsrohr 11 immer in der gleichen Orientierung, bspw. mit dem Boden in Förderrichtung voran, erreichen.

Die Batteriebecher 2 werden einzeln in das obere Ende 19 des Führungsrohrs 11 eingebracht, so dass diese in dem Führungsrohr 11 durch die Inspektionsvorrichtung 9 geführt werden. Bevorzugt ist das Führungsrohr 11 schräg zum Untergrund aufgestellt, so dass sich die Batteriebecher 2 mittels Schwerkraft durch das Führungsrohr 11 bewegen. Jeder Batteriebecher 2 wird an einem oder mehreren Strahlungsmitteln vorbeigeführt. Die Strahlungsmittel geben Strahlung in Form von sichtbarem Licht in Richtung des geführten Batteriebechers 2 ab. Die Strahlung trifft auf den Batteriebecher 2 und wird von diesem zumindest teilweise als Reflexionsstrahlung reflektiert. Die Sensoreinrichtung, bevorzugt in Form eines oder mehrerer Kamerasysteme, empfängt die Reflexionsstrahlung und wandelt diese in Bilddaten um. Die Bilddaten werden dann mittels einer Auswerteeinrichtung ausgewertet. Bevorzugt erfolgt die Auswertung computergestützt mithilfe einer entsprechenden Software.

Basierend auf der Auswertung der Bilddaten beurteilt die Auswerteeinrichtung, ob der jeweilige Batteriebecher 2 herstellungsbedingte Fehlstellen wie bspw. parabolische Aufbrüche, Löcher oder sonstige Beschädigungen aufweist. Weist der inspizierte Batteriebecher 2 Fehlstellen auf, wird er automatisch, beispielsweise über eine mechanische Weiche am unteren Ende 20 des Führungsrohrs 11, von den übrigen Batteriebechern 2 getrennt und in dem Behältnis 16 gesammelt. Weist der inspizierte Batteriebecher 2 keine Fehlstellen auf, so wird er in Kartonage verbracht, die auf der Rollenbahn 10, angrenzend an die Inspektionsvorrichtung 9 angeordnet ist.

### Bezugszeichenliste

- 1: Produktionsanlage
- 2: Batteriebecher
- 3: Tiefziehpresse
- 4: Waschanlage
- 5: Zwischenpuffer
- 6: Separierungseinrichtung
- 7: Zuführsystem
- 8: Förderband
- 9: Inspektionsvorrichtung
- 10: Rollenbahn
- 11: Führungsrohr
- d_{F}: Innendurchmesser (Führungsrohr)
- D_{F}: Außendurchmesser (Führungsrohr)
- D_{B}: Außendurchmesser (Batteriebecher)
- 12: Längsachse
- 13: Aufnahme
- 14: Anschläge (Aufnahme)
- 15: Gehäuse (Inspektionsvorrichtung)
- 16: Behältnis
- 17: Mechanismus
- 18: Griff
- 19: erstes Ende (Führungsrohr)
- 20: zweites Ende (Führungsrohr)
- 21: Fördereinrichtung

## Patentansprüche

1. Inspektionsvorrichtung zur Inspektion von zylindrischen Metallformteilen (2) umfassend,
eine Führung zum Führen der zu inspizierenden Metallformteile (2) durch die Inspektionsvorrichtung (9), Strahlungsmittel, die ausgebildet sind, Strahlung in Richtung der geführten Metallformteile (2) abzugeben, so dass die Strahlung auf jedes der Metallformteile (2) trifft und von diesem zumindest teilweise als Reflexionsstrahlung reflektiert wird,
eine Sensoreinrichtung, die ausgebildet ist, die Reflexionsstrahlung zu empfangen und zu Bilddaten umzuwandeln, und
eine Auswerteeinrichtung, die ausgebildet ist, basierend auf einer Auswertung der Bilddaten zu beurteilen, ob das jeweilige Metallformteil (2) herstellungsbedingte Fehlstellen aufweist,
**dadurch gekennzeichnet,**
**dass** die Führung ein Führungsrohr (11) ist, das ausgebildet ist, die zu inspizierende Metallformteile (2) aufzunehmen, so dass diese in dem Führungsrohr (11) durch die Inspektionsvorrichtung (9) geführt werden,
**dass** das Führungsrohr (11) einen Transmissionsgrad für die Strahlung von 80 % bis 95 % aufweist, gemessen bei einer Wanddicke des Führungsrohrs (11) von 2 mm und für Strahlung aus einem Wellenlängenbereich von 400 nm bis 800 nm und,
**dass** das Führungsrohr (11) einen mittleren linearen thermischen Ausdehnungskoeffizient nach ISO 7991 von höchstens 5×10⁻⁶/K hat.

2. Inspektionsvorrichtung nach Anspruch 1, **dadurch gekennzeichnet, dass** das Führungsrohr (11) einen Transmissionsgrad von 80 % bis 95 % aufweist, gemessen bei einer Wanddicke des Führungsrohrs (11) von 4 mm und für Strahlung aus einem Wellenlängenbereich von 400 nm bis 800 nm.

3. Inspektionsvorrichtung nach einem der vorhergehenden Ansprüche, **dadurch gekennzeichnet, dass** das Führungsrohr (11) eine Knoop'sche Härte HK_{0.1/20} gemäß ISO 9385 von 200 bis 700 aufweist.

4. Inspektionsvorrichtung nach einem der vorhergehenden Ansprüche, **dadurch gekennzeichnet, dass** das Führungsrohr (11) aus Borosilicatglas hergestellt ist.

5. Inspektionsvorrichtung nach Anspruch 4, **dadurch gekennzeichnet, dass** das Borosilicatglas ein erdalkalifreies Borosilicatglas ist.

6. Inspektionsvorrichtung nach einem der vorhergehenden Ansprüche, **dadurch gekennzeichnet, dass** das Führungsrohr (11) einen Innendurchmesser (d_{F}) und die zylindrischen Metallformteile jeweils einen Außendurchmesser (D_{B}) aufweisen, der kleiner ist als der Innendurchmesser (d_{F}) des Führungsrohrs, wobei die Differenz zwischen dem Innendurchmesser des Führungsrohrs (d_{F}) und dem Außendurchmesser (D_{B}) der Metallformteile zwischen 1 mm und 6 mm, bevorzugt zwischen 2 mm und 5 mm, insbesondere bevorzugt zwischen 2,3 mm und 4,8 mm beträgt.

7. Inspektionsvorrichtung nach einem der vorhergehenden Ansprüche, **dadurch gekennzeichnet, dass** sich das Führungsrohr (11) entlang einer Längsachse (12) durch die Inspektionsvorrichtung (9) erstreckt.

8. Inspektionsvorrichtung nach einem der vorhergehenden Ansprüche, **dadurch gekennzeichnet, dass** die Inspektionsvorrichtung (9) eine Aufnahme (13) für das Führungsrohr (11) aufweist, und die Inspektionsvorrichtung (9) derart ausgebildet ist, dass das Führungsrohr (11) aus der Aufnahme (13) entnehmbar und austauschbar ist.

9. Inspektionsvorrichtung nach Anspruch 8, **dadurch gekennzeichnet, dass** das Führungsrohr (11) einen Außendurchmesser (D_{F}) hat und die Aufnahme (13) ausgebildet ist, Führungsrohre (11) mit unterschiedlichen Außendurchmessern (D_{F}) aufzunehmen.

10. Inspektionsvorrichtung nach einem der vorhergehenden Ansprüche, **dadurch gekennzeichnet, dass** das Führungsrohr (11) eine Wanddicke von 2 mm bis 4 mm aufweist.

11. Inspektionsvorrichtung nach einem der vorhergehenden Ansprüche, **dadurch gekennzeichnet, dass** das Führungsrohr (11) derart angeordnet ist, dass sich die Metallformteile (2) mittels Schwerkraft durch das Führungsrohr (9) bewegen.

12. Inspektionsvorrichtung nach einem der Ansprüche 1 bis 10, **dadurch gekennzeichnet, dass** das Führungsrohr (11) mit Druckluft beaufschlagbar und derart ausgebildet ist, dass die Metallformteile (2) mittels Druckluftstößen durch das Führungsrohr (11) bewegt werden.

13. Inspektionsvorrichtung nach einem der vorhergehenden Ansprüche, **dadurch gekennzeichnet, dass** die Metallformteile Edelstahlbecher, insbesondere Batteriebecher (2) sind.

14. Produktionsanlage zum Herstellen von zylindrischen Metallformteilen (2) umfassend,
eine Tiefziehpresse (3) zum Tiefzeihen der Metallformteile (2) aus Rohlingen in mehreren Tiefziehschritten mithilfe eines Stempels und einer Matrize,
eine Waschstation (4) zum Reinigen der tiefgezogenen Metallformteile (2) und
eine Inspektionsvorrichtung (9) nach einem der Ansprüche 1 bis 13 zur Inspektion der gereinigten Metallformteile.

15. Verfahren zum Inspizieren von zylindrischen Metallformteilen (2), umfassend die folgenden Schritte:
Bereitstellen einer Inspektionsvorrichtung nach einem der Anspruch 1 bis 13;
Einbringen eines Metallformteils in das Führungsrohr, so dass dieses in dem Führungsrohr (11) durch die Inspektionsvorrichtung (9) geführt wird;
Aussenden von Strahlung, so dass diese auf das Metallformteil (2) trifft und von diesem zumindest teilweise als Reflexionsstrahlung reflektiert wird;
Empfangen der Reflexionsstrahlung und Umwandlung derselben zu Bilddaten;
Auswerten der Bilddaten und Beurteilen, ob das Metallformteil (2) herstellungsbedingte Fehlstellen aufweist.

## Claims

1. Inspection apparatus for inspecting cylindrical metal forming parts (2) comprising,
a guide for guiding the metal forming parts (2) to be inspected through the inspection apparatus (9),
a radiation device which is adapted to emit radiation in the direction of the guided metal forming parts (2), so that the radiation impinges on each of the metal forming parts (2) and is at least partially reflected therefrom as reflection radiation,
a sensor device adapted to receive the reflection radiation and to convert it into image data, and
an evaluation device which is designed to assess, based on an evaluation of the image data, whether the respective metal forming part (2) has production-related defects,
**characterized in**
**that** the guide is a guide tube (11) which is configured to receive the metal forming parts (2) to be inspected so that they are guided in the guide tube (11) through the inspection apparatus (9),
**that** the guide tube (11) has a transmittance for the radiation of 80% to 95%, measured at a wall thickness of the guide tube (11) of 2 mm and for radiation from a wavelength range of 400 nm to 800 nm and,
**that** the guide tube (11) has an average coefficient of linear thermal expansion according to ISO 7991 of at most 5×10⁻⁶/K.

2. Inspection apparatus according to claim 1, **characterized in that** the guide tube (11) has a transmittance of 80% to 95%, measured at a wall thickness of the guide tube (11) of 4 mm and for radiation from a wavelength range of 400 nm to 800 nm.

3. Inspection apparatus according to any one of the preceding claims, **characterized in that** the guide tube (11) has a Knoop's hardness HK_{0.1/20} according to ISO 9385 of 200 to 700.

4. Inspection apparatus according to any one of the preceding claims, **characterized in that** the guide tube (11) is made of borosilicate glass.

5. Inspection apparatus according to claim 4, **characterized in that** the borosilicate glass is an alkaline-earth free borosilicate glass.

6. Inspection apparatus according to any one of the preceding claims, **characterized in that** the guide tube (11) has an inner diameter (d_{F}) and the cylindrical metal forming parts each have an outer diameter (D_{B}) that is smaller than the inner diameter (d_{F}) of the guide tube, the difference between the inner diameter of the guide tube (d_{F}) and the outer diameter (D_{B}) of the metal forming parts being between 1 mm and 6 mm, preferably between 2 mm and 5 mm, more preferably between 2.3 mm and 4.8 mm.

7. Inspection apparatus according to any one of the preceding claims, **characterized in that** the guide tube (11) extends along a longitudinal axis (12) through the inspection apparatus (9).

8. Inspection apparatus according to any one of the preceding claims, **characterized in that** the inspection apparatus (9) has a receiving portion (13) for the guide tube (11), and the inspection apparatus (9) is designed in such a way that the guide tube (11) can be removed from the receiving portion (13) and can be replaced.

9. Inspection apparatus according to claim 8, **characterized in that** the guide tube (11) has an outer diameter (D_{F}) and the receiving portion (13) is designed to receive guide tubes (11) with different outer diameters (D_{F}).

10. Inspection apparatus according to any one of the preceding claims, **characterized in that** the guide tube (11) has a wall thickness of 2 mm to 4 mm.

11. Inspection apparatus according to any one of the preceding claims, **characterized in that** the guide tube (11) is arranged in such a way that the metal forming parts (2) move through the guide tube (9) by means of gravity.

12. Inspection apparatus according to any one of claims 1 to 10, **characterized in that** the guide tube (11) can be pressurized with compressed air and is designed in such a way that the metal forming parts (2) are moved through the guide tube (11) by shocks of compressed air.

13. Inspection apparatus according to any one of the preceding claims, **characterized in that** the metal forming parts are stainless steel cups, in particular battery cups (2).

14. Production line for manufacturing cylindrical metal forming parts (2) comprising,
a deep-drawing press (3) for deep-drawing the metal forming parts (2) from blanks in multiple deep-drawing steps by means of a punch and a die,
a washing station (4) for cleaning the deep-drawn metal forming parts (2) and
an inspection apparatus (9) according to any one of claims 1 to 13 for inspecting the cleaned metal forming parts.

15. Method for inspecting cylindrical metal forming parts (2), comprising the following steps:
Providing an inspection apparatus according to any one of claims 1 to 13;
Inserting a metal forming part into the guide tube so that it is guided in the guide tube (11) through the inspection apparatus (9);
Emitting radiation so that it impinges on the metal forming part (2) and is at least partially reflected therefrom as reflection radiation;
Receiving the reflection radiation and converting it to image data;
Evaluating the image data and assessing whether the metal forming part (2) has production-related defects.

## Revendications

1. Dispositif d'inspection pour l'inspection de pièces métalliques cylindriques formées (2) comprenant un guidage pour le guidage des pièces métalliques cylindriques formées (2) à inspecter par le dispositif d'inspection (9),
des moyens de rayonnement qui sont réalisés afin d'émettre un rayonnement en direction des pièces métalliques cylindriques formées (2) guidées de sorte que le rayonnement atteigne chacune des pièces métalliques cylindriques formées (2) et soit réfléchi par celle-ci au moins partiellement comme rayonnement réfléchi,
un dispositif capteur qui est réalisé afin de recevoir le rayonnement réfléchi et de le convertir en données d'image et
un dispositif d'évaluation qui est réalisé afin de juger sur la base d'une évaluation des données d'image si la pièce métallique formée (2) respective présente des imperfections dues à la fabrication,
**caractérisé en ce que**
le guidage est un tube de guidage (11) qui est réalisé afin de recevoir les pièces métalliques formées à inspecter (2) de sorte que celles-ci soient guidées dans le tube de guidage (11) par le dispositif d'inspection (9),
le tube de guidage (11) présente un degré de transmission pour le rayonnement de 80 % à 95 %, mesuré pour une épaisseur de paroi du tube de guidage (11) de 2 mm et pour le rayonnement depuis une plage de longueur d'ondes de 400 nm à 800 nm et,
le tube de guidage (11) présente un coefficient de dilatation thermique linéaire médian selon ISO 7991 d'au plus 5×10⁻⁶/K.

2. Dispositif d'inspection selon la revendication 1, **caractérisé en ce que** le tube de guidage (11) présente un degré de transmission de 80 % à 95 %, mesuré pour une épaisseur de paroi du tube de guidage (11) de 4 mm et pour un rayonnement depuis une plage de longueur d'ondes de 400 nm à 800 nm.

3. Dispositif d'inspection selon l'une des revendications précédentes, **caractérisé en ce que** le tube de guidage (11) présente une dureté Knoop HK_{0,1/20} selon ISO 9385 de 200 à 700.

4. Dispositif d'inspection selon l'une des revendications précédentes, **caractérisé en ce que** le tube de guidage (11) est fabriqué en verre au borosilicate.

5. Dispositif d'inspection selon la revendication 4, **caractérisé en ce que** le verre au borosilicate est un verre au borosilicate sans base alcalinoterreuse.

6. Dispositif d'inspection selon l'une des revendications précédentes, **caractérisé en ce que** le tube de guidage (11) présente un diamètre intérieur (d_{F}) et les pièces métalliques cylindriques formées présentent respectivement un diamètre extérieur (D_{B}) qui est inférieur au diamètre intérieur (d_{F}) du tube de guidage, dans lequel la différence entre le diamètre intérieur du tube de guidage (d_{F}) et le diamètre extérieur (D_{B}) des pièces métalliques formées est comprise entre 1 mm et 6 mm, de préférence entre 2 mm et 5 mm, en particulier de préférence entre 2,3 mm et 4,8 mm.

7. Dispositif d'inspection selon l'une des revendications précédentes, **caractérisé en ce que** le tube de guidage (11) s'étend le long d'un axe longitudinal (12) à travers le dispositif d'inspection (9).

8. Dispositif d'inspection selon l'une des revendications précédentes, **caractérisé en ce que** le dispositif d'inspection (9) présente un logement (13) pour le tube de guidage (11), et le dispositif d'inspection (9) est réalisé de telle manière que le tube de guidage (11) puisse être retiré du logement (13) et remplacé.

9. Dispositif d'inspection selon la revendication 8, **caractérisé en ce que** le tube de guidage (11) présente un diamètre extérieur (D_{F}) et le logement (13) est réalisé afin de recevoir des tubes de guidage (11) avec différents diamètres extérieurs (D_{F}).

10. Dispositif d'inspection selon l'une des revendications précédentes, **caractérisé en ce que** le tube de guidage (11) présente une épaisseur de paroi de 2 mm à 4 mm.

11. Dispositif d'inspection selon l'une des revendications précédentes, **caractérisé en ce que** le tube de guidage (11) est agencé de telle manière que les pièces métalliques formées (2) se déplacent par la force de gravité à travers le tube de guidage (9).

12. Dispositif d'inspection selon l'une des revendications 1 à 10, **caractérisé en ce que** le tube de guidage (11) peut être alimenté en air comprimé et est réalisé de telle manière que les pièces métalliques formées (2) soient déplacées au moyen de jets d'air comprimé à travers le tube de guidage (11).

13. Dispositif d'inspection selon l'une des revendications précédentes, **caractérisé en ce que** les pièces métalliques formées sont des boîtiers en acier spécial, en particulier boîtiers de batterie (2).

14. Installation de production pour la fabrication de pièces métalliques cylindriques formées (2) comprenant une presse d'emboutissage (3) pour l'emboutissage profond des pièces métalliques formées (2) à partir d'ébauches dans plusieurs étapes d'emboutissage profond à l'aide d'un poinçon et d'une matrice,
une station de lavage (4) pour le nettoyage des pièces métalliques formées (2) embouties et
un dispositif d'inspection (9) selon l'une des revendications 1 à 13 pour l'inspection des pièces métalliques formées nettoyées.

15. Procédé d'inspection de pièces métalliques cylindriques formées (2) comprenant les étapes suivantes :
la fourniture d'un dispositif d'inspection selon l'une des revendications 1 à 13 ;
l'introduction d'une pièce métallique formée dans le tube de guidage de sorte que celle-ci soit guidée dans le tube de guidage (11) par le dispositif d'inspection (9) ;
l'émission de rayonnement de sorte que celui-ci atteigne la pièce métallique formée et soit réfléchi par celle-ci au moins partiellement comme rayonnement réfléchi ;
la réception du rayonnement réfléchi et la conversion de celui-ci en données d'image ;
l'évaluation des données d'image et le jugement si la pièce métallique formée (2) présente des imperfections dues à la fabrication.
